# EUROPÄISCHE PATENTSCHRIFT

(11) **EP 2 729 440 B1**
(45) Veröffentlichungstag und Bekanntmachung des Hinweises auf die Patenterteilung: **21.12.2016**
(21) Anmeldenummer: 12733112.2
(22) Anmeldetag: 05.07.2012
(51) Int. Cl.: C07C 68/02, C07C 68/08, C07C 69/96, C01B 7/04, C01B 7/07, C25B 1/26

(54) **VERFAHREN ZUR HERSTELLUNG VON DIARYLCARBONATEN**
METHOD FOR PRODUCING DIARYL CARBONATES
PROCÉDÉ DE PRODUCTION DE DIARYLCARBONATES

(30) Priorität: 08.07.2011 EP 11173304
(43) Veröffentlichungstag der Anmeldung: 14.05.2014
(73) Patentinhaber: Covestro Deutschland AG, 51373 Leverkusen (DE)
(72) Erfinder: OOMS, Pieter, 47800 Krefeld (DE); RISSE, Friedhelm, 50739 Köln (DE); DÜX, Andre, 53332 Bornheim (DE); BUCHALY, Carsten, 40233 Düsseldorf (DE); LEIBERICH, Ricarda, 63263 Neu-Isenburg (DE); RONGE, Georg, 40549 Düsseldorf (DE); KRÄMER, Korbinian, 50733 Köln (DE); SCHELLEN, Ralph, 41541 Dormagen (DE); KÖHLER, Karl-Heinz, 52078 Aachen-Brand (DE); VANDEN EYNDE, Johan, 9052 Zwijnaarde (BE)
(74) Vertreter: Levpat
(86) Internationale Anmeldenummer: PCT/EP2012/063140
(87) Internationale Veröffentlichungsnummer: WO 2013/007597

(56) Entgegenhaltungen:
- EP-A2- 1 112 997
- DE-A1- 10 260 084

## Beschreibung

Gegenstand der Erfindung ist ein Verfahren zur Herstellung von Diarylcarbonaten aus Monohydroxy-aromaten und Phosgen in Gegenwart definierter Mengen an ausgewählten Katalysatoren, die im flüssigen Reaktionsgemisch homogen löslich sind. Insbesondere ist es ein Verfahren zur Herstellung von Diarylcarbonat, bevorzugt Diphenylcarbonat (DPC) aus Phenol und Phosgen zumindest teilweise in flüssiger Phase ohne Verwendung zusätzlicher Lösungsmittel, wobei der bei der Reaktion gewonnene Chlorwasserstoff weitgehend frei von Phosgen ist. Das Verfahren wird bevorzugt bei Temperaturen oberhalb von 80°C durchgeführt, um Abscheidungen von gebildetem DPC in fester Form zu vermeiden. Die Umsetzung der Edukte kann sowohl bei Normaldruck oder leicht vermindertem Druck als auch bei erhöhten Drücken von bis zu 50 bar (a), bei denen auch das Phosgen in kondensierter Phase vorliegt, durchgeführt werden. Der bei der Reaktion gewonnene Chlorwasserstoff wird mehreren Reinigungsschritten unterzogen, die so ausgewählt sind, dass der Chlorkohlenwasserstoffstrom für eine Vielzahl weiterer Verwendungsmöglichkeiten geeignet ist, insbesondere für elektrochemische oder thermische Oxidationen zu Chlor. Dieses so gewonnene Chlor kann zur Phosgenherstellung mit Kohlenmonoxid verwendet werden; das erhaltene Phosgen kann im erfindungsgemäßen Verfahren eingesetzt werden. Das unter den Reaktionsbedingungen flüssige Endprodukt wird bevorzugt in mehreren Destillationsschritten von Nebenprodukten gereinigt und weist einen Gehalt von mehr als 99,8% DPC auf. Der in der Reaktion verwendete Katalysator kann dabei so aufgearbeitet werden, dass er zumindest teilweise in die Reaktion zurückgeführt werden kann.

Verfahren zur Herstellung von reinen Diarylcarbonaten aus Monohydroxy-aromaten und Phosgen sind bekannt.

Die Herstellung von Diarylcarbonaten (wie z. B. Diphenylcarbonat) erfolgt üblicherweise durch ein kontinuierliches Verfahren, durch Herstellung von Phosgen und anschließende Reaktion von Monophenolen und Phosgen in einem inerten Lösungsmittel in Gegenwart von Alkali und einem Stickstoffkatalysator in der Grenzfläche.

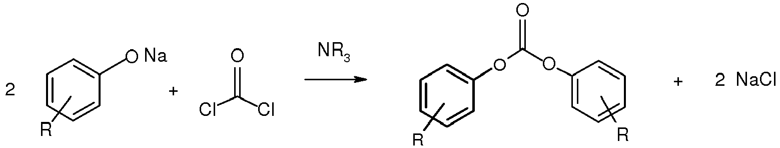

Die Herstellung von Diarylcarbonaten z.B. durch den Phasengrenzflächenprozess ist prinzipiell in der Literatur beschrieben, siehe z.B. in Chemistry and Physics of Polycarbonates, Polymer Reviews, H. Schnell, Vol. 9, John Wiley and Sons, Inc. (1964), S. 50/51.

Dabei werden nach dem Phasengrenzflächen-Verfahren die in Lösemitteln und Wasser gelösten Edukte miteinander umgesetzt. Der Nachteil dieser Verfahren besteht in der destillativen Abtrennung des Diarylcarbonats vom Lösemittel und dessen Wiederaufarbeitung, sowie in der Kochsalz-haltigen wässrigen Phase als Zwangsanfall-Produkt, für die es nur begrenzte Verwendungsmöglichkeiten gibt und die gegebenenfalls sehr aufwendige Aufarbeitungsschritte erfordert.

Daher wurden Verfahren zur sogenannten Direkt-Phosgenierung von Monohydroxy-aromaten entwickelt, bei denen die Edukte Phosgen und Monohydroxy-aromat in der Schmelze in Gegenwart von Katalysatoren, jedoch ohne Verwendung von Lösemitteln, zu Diarylcarbonaten und Chlorwasserstoff umgesetzt werden. Dieses Verfahren hat jedoch den Nachteil, dass HCl enthaltend weitere Nebenkomponenten wie Phosgen erhalten wird, das in dieser Reinheit nicht wiedereinsetzbar ist.

So wird beispielsweise in der US2,362,865 (A) ein Verfahren zur Herstellung von Diarylcarbonaten durch Direkt-Phosgenierung von Monophenolen bei Temperaturen von 170°C bis 250°C unter Verwendung von Al- oder Ti-Phenolaten beschrieben. Was die Reinigung des entstehenden Chlorwasserstoffs von begleitenden Restmengen an Phosgen betrifft, so werden hier zwar Maßnahmen gegebenenfalls zur Wäsche des rohen Chlorwasserstoffs mit Benzol angeführt, aber anschließend erfolgt die Absorption des Chlorwasserstoffs in Wasser unter Bildung von Salzsäure oder die Absorption in Alkalilauge unter Bildung von Kochsalzlösung. Dadurch wird allerdings kein gereinigter gasförmiger Chlorwasserstoff erhalten.

Sowohl in der zum Zeitpunkt dieser Anmeldung unveröffentlichten EP 10158364.9 (EP 2371806 A1) als auch in der unveröffentlichten EP 10158446.4 (EP 2371807 A1) werden ebenfalls Verfahren zur Herstellung von Diarylcarbonaten durch Direkt-Phosgenierung von Monophenolen bei Temperaturen von 20°C bis 240°C unter Verwendung von Metall-Halogeniden oder Metall-Phenolaten beschrieben.

Die Reinigung des entstehenden Chlorwasserstoffs von Spuren an Phosgen wird, gemäß bekanntem Stand der Technik, abgesehen von dem Auskondensieren des Phosgens und weiterer flüchtiger Bestandteile in Kühlfallen bei tieferen Temperaturen, zusätzlich oder alternativ durch Waschen mit kalten Lösemitteln, wie z.B. Chlorbenzolen, bei Temperaturen von -15°C bis -45°C durchgeführt. Diese Maßnahme bedingt die Einführung eines zusätzlichen Stoff-Kreislaufs, was zur Kontamination des Chlorwasserstoffs mit Chlorkohlenwasserstoffe führt, der für eine nachfolgende Verwendung, beispielsweise einer Deacon-Oxidation, hinderlich ist und daher in einem zusätzlichen Schritt wieder entfernt werden muss. Diese Vorgehensweise ist sehr aufwändig und daher zu vermeiden ist, und bedingt einen weiteren sehr hohen Energie-Aufwand und beeinträchtigt damit erheblich die Energie- und Kosten-Effizienz.

In der EP 1 234 845 A wird ebenfalls die Umsetzung von Monophenolen in der Schmelze bei Temperaturen von 120°C bis 190°C mit einem besonders reinen Phosgen beschrieben. Als Katalysatoren werden Stickstoff-haltige Verbindungen, wie z.B. Pyridin in Mengen von 0,1 bis 10mol% bezogen auf eingesetztes Monophenol verwendet. Die beschriebene Aufarbeitung der gasförmigen Reaktionsprodukte führt ferner nicht zu einem gereinigten gasförmigen Chlorwasserstoff als verwertbares Endprodukt, sondern zu Salzsäure- oder Kochsalzlösungen durch Absorption in Wasser bzw. in Alkalilauge.

In der EP 1 112 997 A2 wird die Umsetzung von Monophenolen in der Schmelze mit Phosgen bei Temperaturen von 80°C bis 180°C, sowie die Oxidation des entstandenen Chlorwasserstoffs zu Chlor beschrieben. Der Chlorwasserstoff wird von störenden Nebenprodukten durch Kontakt mit Adsorbentien, wie z.B. Aktivkohle, gereinigt, bevor er in Wasser zu Salzsäure gelöst und elektrolysiert wird oder gasförmig einer thermischen Oxidation unterworfen wird. In dieser Schrift wird lediglich eine Kondensation als mögliche Reinigungsmaßnahme offenbart. Definierte Angaben zu zulässigen Restkonzentrationen von Nebenprodukten wie Phosgen oder ChlorKohlenwasserstoffen im Chlorwasserstoff für den Einsatz in den nachfolgenden Oxidationen zu Chlor fehlen hier.

Darüber hinaus gibt es eine Anzahl weiterer Schutzrechte, wie z.B. WO 2008/114750 A1, JP 2008-231073 A, JP 2009-114195 A, JP 09-278714 A, JP 09-100256 A, in denen die Umsetzung von Monophenolen bei höheren Temperaturen in der Schmelze mit Phosgen in Gegenwart homogen löslicher Stickstoff-haltiger Katalysatoren zu Diarylcarbonaten beschrieben wird.

In der WO 95/25083 A1, sowie in der EP 567 677 A1 werden jeweils im Reaktionsmedium homogen lösliche Phosphorverbindungen, wie z.B. Phosphite oder Phosphine als Katalysatoren in Direkt-Phosgenierungsreaktionen von Monophenolen eingesetzt

In keiner dieser Veröffentlichungen gibt es Hinweise auf das Erfordernis, auf eine besondere Reinheit des Chlorwasserstoffes zu achten oder die Nebenkomponenten im Chlorwasserstoff auf definierte Grenzwerte zu beschränken. Insbesondere gibt es keine Hinweise darauf, dass der Fachmann auf bestimmte Nebenkomponenten, beispielsweise Phosgen, achten soll.

Die aus dem Stand der Technik bekannten Verfahren reichen nicht um hohe Reinheiten der Produkte sicherstellen, die ihrerseits Einsatzstoffe für weitere chemische Prozesse sind, bei denen Nebenprodukte stören würden. Zudem reichen diese darüber hinaus nicht, um den hohen ökonomischen und ökologischen Anforderungen gerecht zu werden. So ist z.B. die für viele Verfahren typische Bildung von wässrigen NaCl-Lösungen nicht akzeptabel, da solche Zwangsanfall-Produkte ökologisch bedenklich sind oder einen relativ hohen Aufwand z.B. durch Aufkonzentrieren und anschließendes Elektrolysieren erfordern. Erstrebenswert dagegen ist die Bildung insbesondere von gasförmigem Chlorwasserstoff, da sich für einen reinen Chlorwasserstoff sehr viele Verwendungsmöglichkeiten anbieten; dazu gehört insbesondere die Recyclisierung von Chlorwasserstoff über die Stufen von Chlor und Phosgen in das Herstell-Verfahren zurück. Solche Recyclisierungen können nach dem Stand der Technik durch Elektrolyse des Chlorwasserstoffs in Form von Salzsäure nach verschiedenen Verfahren vorgenommen werden, wobei das Diaphragma-Verfahren oder die Elektrolyse der Salzsäure mit einer Sauerstoffverzehrkathode als moderne und bedeutende Herstellverfahren für Chlor genannt seien. Diese Elektrolyse-Verfahren erfordern eine ausreichende Reinheit des Chorwasserstoffs, um einen ungestörten Elektrolyseprozess zu gewährleisten. Eine andere Methode zur Herstellung von Chlor aus Chorwasserstoff nach dem Stand der Technik ist das Deacon-Verfahren, wobei Chlorwasserstoff chemisch zu Chlor oxidiert wird. Ein wichtiges Reinheitskriterium für Chlorwasserstoff auch bei der Vermarktung als Verkaufsprodukt ist die weitestgehende Abwesenheit von Phosgen und organischen Verbindungen im Chlorwasserstoff.

Neben den Reinheitskriterien für die Produkte ist auch die Effizienz des Herstellverfahrens ein wichtiges Verfahrensmerkmal. So ist z.B. der Umsatz von Phosgen mit Phenol in der kondensierten homogenen Phase aufgrund der erhöhten Konzentration vom Phosgen im Phenol deutlich höher, als im zweiphasigen Gemisch von gasförmigem Phosgen und flüssigem Phenol. Auch die Temperaturerhöhung wirkt beschleunigend auf die Reaktionsgeschwindigkeit, sodass erhöhte Temperaturen im Bereich von 100°C bis 250°C, vorzugsweise 110°C bis 220°C von Vorteil sein können. Solche Temperaturen wirken allerdings der Löslichkeit von Phosgen in Phenol entgegen, sodass eine Reaktionsführung bei erhöhter Temperatur unter Druck besonders vorteilhaft ist.

Daher stellte sich die technische Aufgabe ein Verfahren zur Herstellung von Diarylcarbonaten nach dem Direkt-Phosgierungs-Verfahren in der Schmelze eines Monophenols unter Verzicht auf zusätzliche Lösemittel bei erhöhter Temperatur und gegebenenfalls bei erhöhtem Druck zu entwickeln, das durch geeignete Maßnahmen zu einem HCl-Strom führt, das sich durch besonders gute Qualität auszeichnet und dennoch mit hoher Wirtschaftlichkeit betrieben wird. Insbesondere bezieht sich die Erfindung auf ein Verfahren zur Herstellung von Diarylcarbonaten, aus dem gasförmiger Chlorwasserstoff mit einem Restgehalt an Phosgen und/oder Chlorkohlensäureestern von weniger als 100 Gew. ppm, vorzugsweise von weniger als 50 Gew. ppm und einem Restgehalt an organischen Verbindungen (insbesondere von Chlorkohlenwasserstoffe) von weniger als 1000 Gew. ppm, vorzugsweise von weniger als 100 Gew. ppm und besonders bevorzugt von weniger als 50 Gew. ppm bereitgestellt wird, wobei diese Reinigungsmethode möglichst wirtschaftlich betrieben werden soll.

Weiterhin spielt das Verwenden von Kühlmittel mit möglichst hohem Temperaturniveau (z. B.. 30°C) eine wichtige wirtschaftliche Rolle. In der Tat steigt der Erzeugungsaufwand von niedrigeren Temperaturniveaus mit sinkender Temperatur, und daher ebenfalls die Erzeugungskosten. Daher ist es von Vorteil, die Menge Kühlmittel mit niedrigem Temperaturniveau (z. B.. -40°C) so weit wie möglich zu minimieren und Kühlmittel mit einem höheren Temperaturniveau (z. B. 6°C und bevorzugt 30°C) verwenden. Die angegebenen Temperaturen dienen hier lediglich der Veranschaulichung.

Organische Verbindungen bedeuten hier Stoffe wie Phenole, Diarylcarbonate, Kohlenmonoxid oder chlorierte Kohlenwasserstoffe, wie z.B. Tetrachlorkohlenstoff. Der hier erhaltene gasförmige Chlorwasserstoff ist insbesondere geeignet als Rohstoff für Oxidationsverfahren zur Herstellung von Chlor. Solche Oxidations-Verfahren sind z.B. elektrochemische Oxidationen durch sogenannte anodische Oxidation in Elektrolysezellen; dazu gehören z.B. die klassischen Amalgam-Elektrolyse-Anlagen, ebenso wie die moderneren Diaphragma-Elektrolysezellen oder die sog. SauerstoffVerzehrkathode. Es gibt darüber hinaus auch chemische Oxidations-Verfahren, wie z.B. den Deacon-Prozess, bei dem gasförmiger Chlorwasserstoff bei hohen Temperaturen beispielsweise an Kupfer-Kontakten zu Chlor oxidiert wird, wobei Wasser als Nebenprodukt entsteht. Der hier erhaltene Chlorwasserstoff kann ebenfalls zur Herstellung von hochreiner Salzsäure durch Auflösen in reinem Wasser verwendet werden. Daher ist ein weiterer Gegenstand der Erfindung die Verwendung des im erfindungsgemäßen Herstell-Verfahren gewonnenen Chlorwasserstoffs für den Einsatz in Verfahren zur Herstellung von Chlor durch Oxidations-Verfahren oder für den Einsatz zur Herstellung von hochreiner Salzsäure. Die Reinheit des eingesetzten Chlorwasserstoffs ist deswegen von Bedeutung, da einige der oben genannten organischen Verbindungen, sowie Chlorkohlensäureester und Phosgen als Katalysator-Gifte wirken und damit z.B. den Deacon-Prozess negativ beeinflussen, oder den Elektrolyseprozess beeinträchtigen. Auch für die Herstellung reiner Salzsäure sind Verunreinigungen wie Phosgen, Chlorkohlensäureester oder Tetrachlorkohlenstoff nicht zu akzeptieren. Bei der Verwendung des erfindungsgemäß gewonnenen Chlorwasserstoffs für Oxidations-Verfahren beziehen sich die oben angegebenen Reinheiten des Chorwasserstoffs auf die für das jeweils unmittelbare Verfahren erforderlichen Reinheiten, unabhängig von irgendwelchen zusätzlichen Chlorwasserstoff-Reinigungsschritten vor diesen jeweiligen Oxidations-Verfahren. Es ist dabei also unerheblich, ob Chlorwasserstoff minderer Qualität in diesen Oxidations-Verfahren eingesetzt und erst durch zusätzliche Schritte vor diesen Verfahren auf die erforderliche Reinheit gebracht wird; entscheidend für die Verwendung des Chlorwasserstoffs ist dessen Reinheit am Eintritt in das jeweilige Oxidationsverfahren.

Die technische Aufgabe konnte überraschenderweise dadurch gelöst werden, dass im Verfahren zur Herstellung von Diarylcarbonaten bestimmte Maßnahmen zur Aufreinigung von Chlorwasserstoff getroffen werden. Die Kombination aus wenigstens zwei Reinigungsmaßnahmen, einer Destillation in Kombination mit einer weiteren Reinigungsmaßnahme, ausgewählt aus der Gruppe bestehend aus Kondensation und Wäsche, bietet eine sehr wirtschaftliche Verfahrensführung, die dennoch zu exzellenten Reinheitsgraden führt, wobei die Reihenfolge der Reinigungsmaßnahmen beliebig ist.

Das erfindungsgemäße Verfahren besteht aus den drei Verfahrens-Abschnitten:
A) Reaktion,
B) Chlorwasserstoff-Aufarbeitung
C) Diarylcarbonat-Aufarbeitung

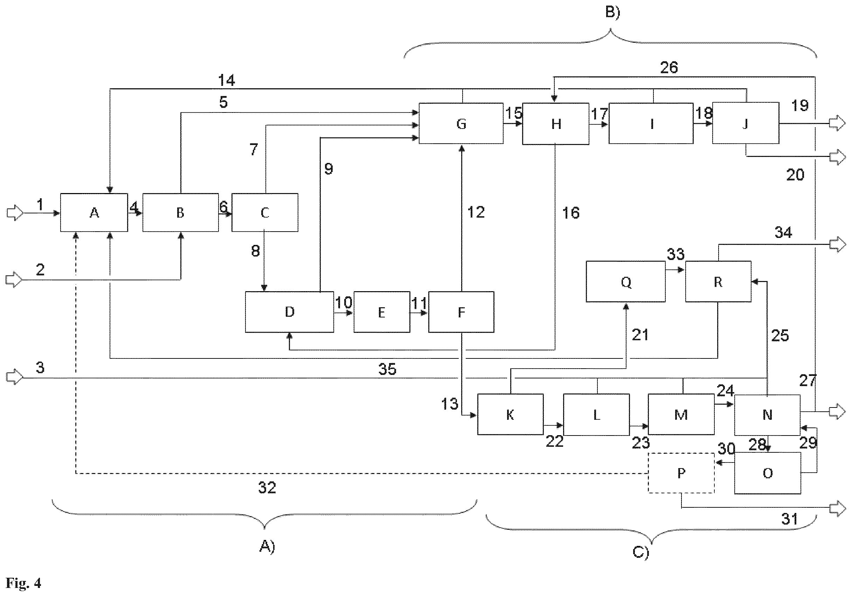

Eine graphische Gesamt-Übersicht gibt die Figur 4, die Erfindung ist jedoch nicht an dieser Verfahrensführung limitiert. (Gesamtverfahren)

In dem Verfahrens-Abschnitt A), Reaktion, werden die Edukte in einem vorgelagerten VerfahrensSchritt in der Weise mit einander vermischt, dass eine weitgehend homogene Lösung von Phosgen im geschmolzenen Monophenol vorliegt; dies kann gegebenenfalls durch Anwendung erhöhter Drücke bei den vorgegebenen Schmelzetemperaturen erfolgen. Der Begriff "Monophenol" wird im Folgenden für alle aromatischen Mono-Hydroxy-Verbindungen benutzt, wobei es sich um ein- oder mehrkernige substituierte oder unsubstituierte aromatische Mono-Hydroxy-Verbindungen handeln kann. Geeignete Monophenole für das erfindungsgemäße Verfahren sind aromatische Verbindungen, die eine OH-Gruppe an einem cyclischen aromatischen Kohlenstoff-Kern gebunden enthalten. Es kann sich dabei um ein- oder mehrkernige aromatische Kohlenstoff-Verbindungen oder ein- oder mehrkernige heterocyclische Verbindungen mit beispielsweise ein oder mehreren Stickstoff- oder Phosphor-Atomen handeln. Diese aromatischen Verbindungen können weiterhin einen oder mehrere Substituenten an verschiedenen Positionen des aromatischen Kerns enthalten.

Im Rahmen der Erfindung geeignete aromatische(n) Hydroxyverbindung(en) (Monophenole) die in Schritt (h) verwendet werden sind bevorzugt solche der allgemeinen Formel (I) 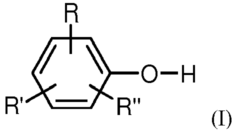 worin R, R' und R" unabhängig voneinander Wasserstoff, Halogen oder ein verzweigter oder unverzweigter C₁- bis C₉-Alkylrest oder Alkoxycarbonylrest sein können.
Solche aromatischen Monophenole sind beispielsweise: Phenol, o-, m- oder p-Kresol, auch als Gemisch der Kresole, Dimethylphenol, auch als Gemisch, wobei die Stellung der Methylgruppen am Phenolring beliebig sein kann, z.B. 2,4-, 2,6-, oder 3,4-Dimethylphenol, o-, m- oder p-Chlorphenol, 2,4-Dichlorphenol, p-Bromphenol und 2,4,6-Tribromphenol, o-, m- oder p-Ethylphenol, o-, m- oder p-n-Propylphenol, 4-iso-Propylphenol, 4-n-Butyl-phenol, 4-iso-Butylphenol, 4-tert-Butylphenol, 4-n-Pentylphenol, 4-n-Hexylphenol, 4-n-Octylphenol, 4-iso-Octylphenol, 4-n-Nonylphenol, 4-iso-Nonylphenol, o-, m- oder p-Methoxyphenol, 4-Cyclohexylphenol, 4-(1-Methyl-1-phenylethyl)-phenol, Biphenyl-4-ol, 1-Naphthol, 2-1-Naphthol, 4-(1-Naphthyl)phenol, 4-(2-Naphthyyl)-phenol, 4-Phenoxyphenol, 3-Pentadecylphenol, 4-Tritylphenol, Methylsalicylsäure, Ethylsalicylsäure, n-Propylsalicylsäure, iso-Propylsalicylsäure, n-Butylsalicylsäure, iso-Butylsalicylsäure, tert-Butylsalicylsäure, Phenylsalicylsäure, Benzylsalicylsäure und Salicylsäuremethylester.

Bevorzugte Monophenole sind Phenol, 4-tert-Butylphenol, Biphenyl-4-ol und 4-(1-Methyl-1-phenylethyl)-phenol.

Besonders bevorzugt ist Phenol.

Die eingesetzten Monophenole sollten eine Reinheit von mindestens 99,90 Gew% aufweisen. Bevorzugt enthalten die Edukte weniger als 300 Gew. ppm Wasser da die Anwesenheit von Wasser die Korrosion der Materialien begünstigt.

Das hier eingesetzte Monophenol kann neben dem von außen in den Gesamtprozeß eingeführte Phenol, dem sogenannten Frischphenol aus Vorratstanks, auch rückgeführtes Monophenol aus Kondensatströmen der Verfahrensschritte B) und C) oder aus Wasch-Flüssigkeitsströmen des Verfahrensschritts B) enthalten. Derartig rückgeführtes Monophenol kann Nebenprodukte aus dem Verfahren enthalten, wie z.B. Restmengen an Diarylcarbonat, Chlorwasserstoff oder Aryl-chlorkohlensäureester, die für die Reaktion unschädlich sind. In der eingesetzten Mischung der Edukte liegt das Monophenol vorzugsweise in mehr als der stöchiometrisch erforderlichen Menge bezogen auf Phosgen vor. Das molare Verhältnis von Monophenol zu Phosgen kann von 1,5:1 bis 4:1 variieren, bevorzugt ist ein Molverhältnis von 2:1 bis 3:1, besonders bevorzugt ist ein Molverhältnis von 2,5:1 bis 3:1.

Das eingesetzte Phosgen sollte zur Vermeidung von unerwünschten Nebenprodukten in den Endprodukten des Herstell-Verfahrens eine Reinheit von wenigstens 99,80 Gew%, vorzugsweise von 99,96 Gew% aufweisen; der Gehalt an Tetrachlorkohlenstoff sollte kleiner als 50 Gew. ppm, vorzugsweise kleiner als 15 Gew. ppm sein.

Geeignete Katalysatoren für das erfindungsgemäße Verfahren sind solche, die sich unter den Reaktionsbedingungen im Reaktionsgemisch ohne Zuhilfenahme zusätzlicher Lösemittel homogen lösen. Solche Katalysatoren sind z.B. Metallsalze, Amine, heterocyclische Stickstoffverbindungen, Phosphine oder Phosphor-Sauerstoff-Verbindungen. Bevorzugte Katalysatoren sind Metallsalze, wie z.B. Aluminiumhalogenide, Zirkonhalogenide oder Titanhalogenide, oder heterocyclische Stickstoff-Verbindungen, wie z.B. Chinolin, Chinazolin oder Pyridin. Besonders bevorzugte Katalysatoren sind Titan-tetrachlorid, Aluminium-trichlorid, Zirkon-tetrachlorid oder Pyridin. Ganz besonders bevorzugt ist Titan-tetrachlorid und Aluminium-Trichlorid. Die Metall-Chloride wandeln sich unter den Reaktionsbedingungen relativ schnell ganz oder teilweise in die entsprechenden Metall-Phenolate, wie z.B. Titan-tetraphenolat um, die dann die homogen gelöste aktive Katalysator-Komponente darstellen. Die erfindungsgemäß verwendeten Katalysatoren können in Mengen von 0,001 mol% bis 10 mol% , vorzugsweise in Mengen von 0,01 mol% bis 5 mol% und besonders bevorzugt in Mengen von 0,05 mol% bis 2 mol% bezogen auf vorhandenes Monophenol eingesetzt werden.

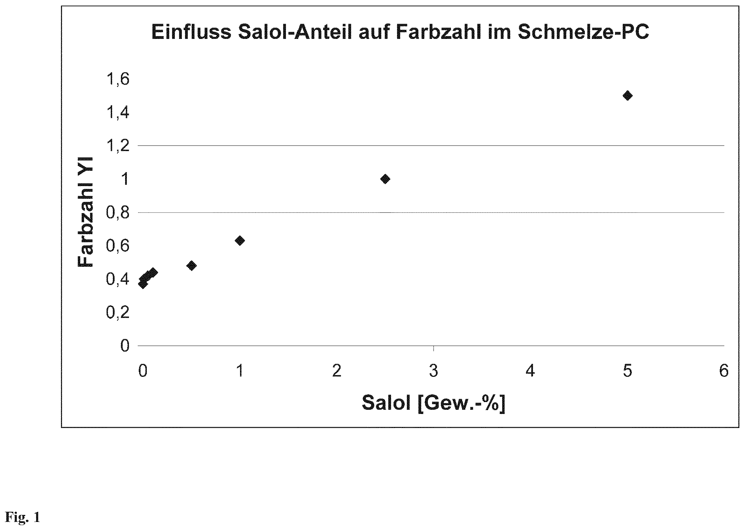

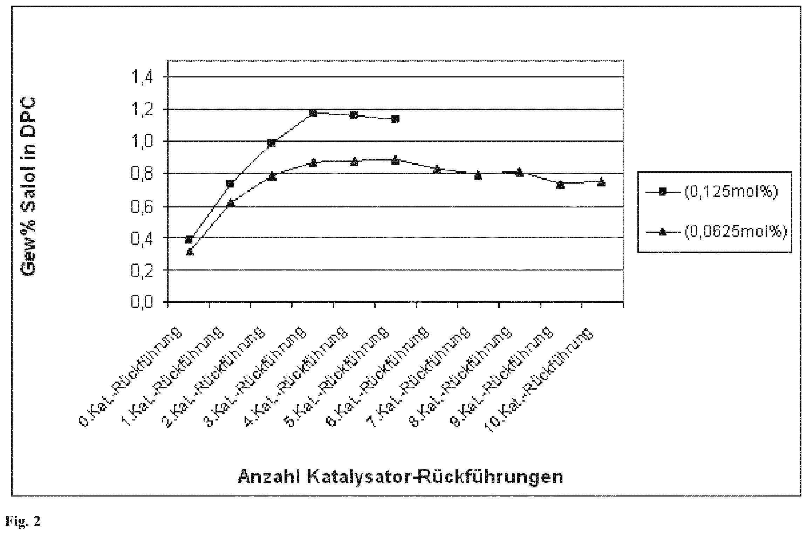

Überraschenderweise wurde gefunden, dass der Gehalt an o-Hydroxy-Benzoesäure-derivaten im Diarylcarbonat auch bei wiederholtem Einsetzen des verwendeten Katalysators, der am Verfahrensende aus dem Prozess zurückgewonnen wird, nicht weiter steigen, sondern stagnieren (Plateau in Fig. 2). Eine solche Rückgewinnung des Katalysators kann beispielsweise durch Rückführung des Sumpfproduktes aus der letzten Diarylcarbonat-Destillationsstufe erfolgen. Dieser wiederholte Einsatz von zurückgewonnem Katalysator im erfindungsgemäßen Diarylcarbonat-Herstellverfahren kann sowohl batch-weise als auch in kontinuierlich arbeitenden Verfahren erfolgen. Die Fig. 2 zeigt am Beispiel der Herstellung von Diphenylcarbonat in Gegenwart von Titantetrachlorid als Katalysator die Bildung von Salol als Nebenprodukt in Abhängigkeit von der Anzahl der wiederholten Einsätze von zurückgewonnenem Katalysator in die Reaktion. Umesterungsfähige Nebenprodukte, wie z.B. Salicylsäurephenylester (Salol) können als artfremde Endgruppen in die Polymerkette eingebaut werden und unvorhersehbare Nebenwirkungen haben. Beispielsweise hat Salol einen direkten Einfluss auf die Qualität des daraus hergestellten Polycarbonats, wie aus Fig. 1 zu entnehmen ist. Dabei wird deutlich, dass unerwarteterweise bei wiederholtem Einsatz des Katalysators die Entstehung von Salol keine lineare Abhängigkeit zwischen Rückführung und Verunreinigung erfolgt, sondern ein Plateau erreicht wird, bei dem die Konzentration an o-Hydroxy-Benzoesäure-derivaten nicht mehr ansteigt.

Die Katalysatoren werden vorzugsweise als Lösung in der Monophenol-Schmelze eingesetzt. Solche Lösungen können auch Katalysatormengen enthalten die aus dem Verfahrensabschnitt C) der destillativen Diarylcarbonat-Aufarbeitung als Diarylcarbonat-Sumpfprodukt anfallen und dann mit oder ohne gesonderte Katalysator-Aufarbeitung in die Reaktion als Verfahrensabschnitt A) zurückgeführt werden. Eine Katalysator-Aufarbeitung ist daher für die Rückführung des Katalysators in den Verfahrensabschnitt A) nicht zwangsweise erforderlich aber durchaus möglich.

Die Zugabe der Katalysatoren erfolgt frühestens nach erfolgter vollständiger Durchmischung der Edukte vorzugsweise in dem Reaktor, um eine vorzeitige Reaktion der Edukte beim Vermischen und damit eine vorzeitige Chlorwasserstoff-Entwicklung in einem ungeeigneten Verfahrensabschnitt zu vermeiden.

Eine derartige Rückführung von Katalysatormengen aus dem Verfahrensabschnitt C) kann beliebig häufig vorgenommen werden; bei sogennanten Konti-Verfahren kann vorzugsweise eine Teilmenge des Katalysators kontinuierlich zurückgeführt werden, während eine Restmenge als Destillations-Sumpf aus dem Verfahrens-Kreislauf ausgeschleust wird. Bei Bedarf kann frischer Katalysator der zurückgeführten Katalysatormenge hinzugefügt werden. In einer bevorzugten Ausführungsform werden wenigstens 25 Gew.% des Katalysators zurückgeführt, besonders bevorzugt wenigstens 50 Gew.%, ganz besonders bevorzugt wenigstens 75 Gew.% und insbesondere ganz besonders bevorzugt wenigstens 85 Gew.%. In einer bevorzugten Ausführungsform werden jedoch maximal 99 Gew.% des Katalsyators zurückgeführt, bevorzugt 95 Gew.%

Die Konzentration des Nebenprodukts im Diarylcarbonat strebt überraschenderweise nach mehrmalig wiederholter oder längerer Katalysator-Rückführung Grenzwerten entgegen, sodass bei lang anhaltender Rückführung des Katalysators kaum noch eine relative weitere Erhöhung der Nebenprodukt-Konzentration im Diarylcarbonat zu beobachten ist.

Falls eine Isolierung des Katalysators erwünscht ist, so ist sie abhängig von der Art des eingesetzten Katalysators und kann im Fall der Verwendung von Titan-tetrachlorid z.B. eine Phosgenierung des im Destillations-Sumpf gebildeten Titan-tetra-Monophenolats bzw. Titan-Salolat zu Titan-tetrachlorid bedeuten. Auch Titanate mit Salol sind katalytisch aktiv. Bei Einsatz von Pyridin wäre das entstandene Pyridin-Hydrochlorid mit Basen in das freie Pyridin zu überführen.

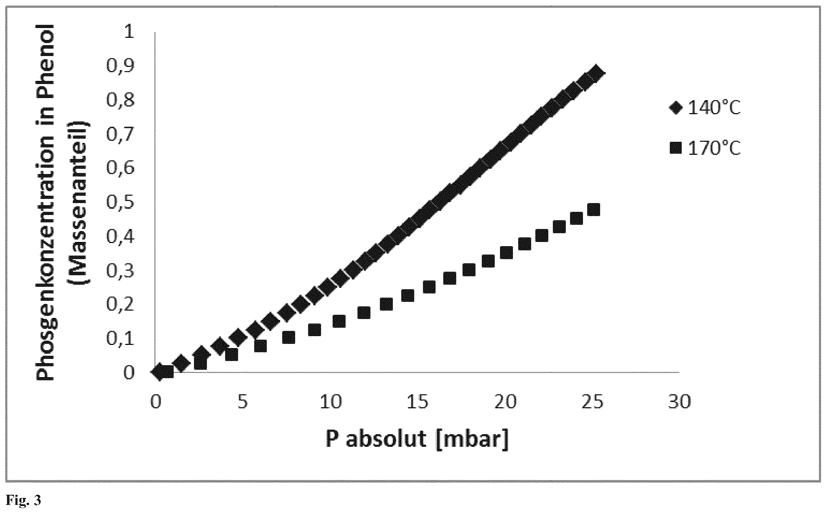

Die Edukte Monophenol und Phosgen werden in den oben angegebenen molaren Verhältnissen bzw. in den oben genannten bevorzugten molaren Verhältnissen mit einander gemischt, wobei das Monophenol stets als Schmelze vorliegt und das Phosgen je nach vorherrschendem Druck gasförmig oder flüssig ist. Bei Normaldruck und Temperaturen oberhalb von 60°C liegt weitgehend ein zweiphasiges Gas-Flüssigkeitsgemisch vor, da die Löslichkeit von Phosgen auch in Monophenolen, ebenso wie in Diarylcarbonaten bei zunehmender Temperatur abnimmt, wie es in Fig. 3 für die Löslichkeit von Phosgen in Phenol veranschaulicht wurde.

Daher müssen Gemische aus geschmolzenen Monophenolen und Phosgen in der Reaktionsphase sehr intensiv gemischt und redispergiert werden, um über eine ausreichende Erneuerung der Phasengrenzflächen einen hinreichenden Umsatz der Edukte zu gewährleisten. Deshalb werden die Edukte bei erhöhter Temperatur unter Normaldruck, vorzugsweise unter erhöhtem Druck bis zu 50 bar (a) - wobei (a) den Absolutdruck bedeutet - besonders bevorzugt bei erhöhtem Druck bis 25 bar (a) und ganz besonders bevorzugt bei Drücken von 8 bis 25 bar (a) mit einander vermischt und zur Reaktion gebracht. Die Temperatur in der Mischzone sollte mindestens die Schmelztemperatur des Monophenols betragen, vorteilhaft aber eine Reaktionstemperatur im Bereich von 100°C bis 250°C erreichen.

Nach der weitgehend vollendeten Mischung der Edukte wird dem Gemisch vorzugsweise einer der oben genannten Katalysatoren vorzugsweise in der bevorzugten Menge als Lösung im Monophenol hinzugesetzt. Da bei den oben genannten Temperaturen und Drücken die katalysierte Umsetzung von Monophenol mit Phosgen zum Aryl-chlorkohlensäureester als Zwischenprodukt sehr rasch unter Abspaltung von gasförmigem Chlorwasserstoff verläuft, kann die Reaktion bevorzugt mehrstufig durchgeführt werden. Die Reaktion kann unter adiabatischen Bedingungen geführt werden, da sie nur eine leichte Wärmetönung aufweist. In einer ersten Stufe, dem sogenannten Hauptreaktor, entstehen insbesondere bei erhöhtem Druck und bevorzugt bei Temperaturen von 120°C bis 230°C, besonders bevorzugt bei Temperaturen von 130°C bis 210°C und für die Herstellung von Diphenylcarbonat ganz besonders bevorzugt bei einer Temperatur von 160°C bis 180°C und bei Reaktor-Verweilzeit von 5 bis 60 min , vorzugsweise von 5 bis 10 min überwiegend Aryl-chlorkohlensäureester neben bereits weiterreagiertem Diarylcarbonat. In einer zweiten Stufe reagiert in einem sogenannten Nachreaktor der Aryl-chlorkohlensäureester bei etwas höheren Temperaturen von vorzugsweise 170°C bis 250°C, besonders bevorzugt von 190°C bis 230°C und ganz besonders bevorzugt von 200°C bis 210°C bei Reaktor-Verweilzeiten von 5 bis 60 min , vorzugsweise von 5 bis 20 min mit noch vorhandenem Monophenol zum Diarylcarbonat. Dabei kann der Druck in der zweiten Stufe im sogenannten Nachreaktor auch auf 2 bis 20 bar abgesenkt werden. Eine derartige Absenkung des Druckes kann vorteilhaft in einer sogenannten Flash-Stufe vorgenommen werden, in der als Folge der Druck-Absenkung das im Hauptreaktor entstandene Chlorwasserstoff-Gas besonders gut aus der Reaktions-Schmelze abgetrennt werden kann. Auch hinter der zweiten Reaktions-Stufe im Nachreaktor kann sich optional eine Flash-Stufe zur Abtrennung der Restmengen an Chlorwasserstoff befinden. Sie kann optional auch in die erste Destillationskolonne des nachfolgen Verfahrensabschnitts C), der destillativen Diarylcarbonat-Aufarbeitung integriert sein und dort die Trennung von Gas- und Flüssig-Phase beschleunigen.

Als Reaktoren für die Umsetzung der Edukte unter den angegebenen Reaktions-Bedingungen sind vorzugsweise Konti-Reaktoren gut geeignet, möglich ist aber auch die Verwendung von Rührkesseln als batch-Reaktoren. Besonders gut geeignete Konti-Reaktoren sind z.B. Rührkesselkaskaden, Blasensäulen-Kolonnen, Boden-Kolonnen, Füllkörper-Kolonnen oder Kolonnen mit feststehenden Einbauten zur Durchmischung des Reaktionsmediums oder Reaktions-Destillations-Kolonnen.

Solche Kolonnen können auch mit einander kombiniert sein, wie z.B. eine Blasensäulen-Kolonne mit einer aufgesetzten Rektifikations-Kolonne, wobei abweichend von der oben beschriebenen Mischung der Edukte, die Edukte getrennt an unterschiedlichen Stellen der Kolonnen-Kombination eingeführt werden können. So kann z.B. in der oben genannten Kolonnen-Kombination das Phosgen in die untere Blasensäulen-Kolonne und das Monophenol zusammen mit dem Katalysator in die obere Rektifikations-Kolonne mit ca. 10 theoretischen Böden eingeführt werden. Das entstandene Diarylcarbonat wird aus der Blasensäulenkolonne entnommen. Der gebildete Chlorwasserstoff wird am Kopf der Rektifikationskolonne entnommen.

Eine entsprechend getrennte Dosierung der Edukte kann auch in einer Reaktions-Destillations-Kolonne derart erfolgen, dass das Phosgen in der Mitte der Kolonne eingeführt wird und dass das Monophenol zusammen mit Katalysator am Kopf der Kolonne eingebracht wird. Das Reaktionsgemisch wird dem Kolonnen-Sumpf entnommen. Solche Kolonnen können mindestens 5, vorzugsweise ca. 20 Böden aufweisen.

In einer weiteren optionalen Ausführungsform der Reaktoren können die Edukte in einem Hauptreaktor bei Drücken von 1 bis 25 bar (a) bei ausreichend hoher, gegebenenfalls längerer Verweilzeit aber geringeren Temperaturen im unteren Teil des Reaktors von vorzugsweise 120°C bis 190°C, besonders bevorzugt von 160°C bis 180°C vollständig umgesetzt werden. Im oberen Teil des Reaktors ist eine zusätzliche Beheizung erforderlich, um dort etwas höhere Temperaturen bis 250°C, vorzugsweise bis 230°C zu realisieren. Die weitgehende Entgasung des Reaktionsgemischs und Abtrennung der Leichtsieder kann anschließend durch eine Flash-Verdampfung oder eine andere Entgasungstechnik erfolgen.

Besonders bevorzugt sind Blasensäulen-Kolonnen, die mit dem Edukt-Gemisch wie oben beschrieben von unten nach oben durchströmt werden. Dabei werden am Kolonnen-Kopf der Blasensäulen-Kolonne der gasförmige Chlorwasserstoff und am oberen Ende des Kolonnen-Schafts das Reaktionsgemisch entnommen. Dieses wird der nächsten Blasensäulen-Kolonne, die als Nachreaktor fungiert, über den Kolonnen-Boden zugeführt. Aus der letzten Blasensäulen-Kolonne wird das vollständig ausreagierte Reaktionsgemisch am Ende eines Verweil-Reaktors entnommen und dem nachfolgenden Verfahrensabschnitt C), der destillativen Diarylcarbonat-Aufarbeitung zugeführt. Das jeweils am Kopf der Blasensäulen-Kolonnen entnommene Chlorwasserstoff-Gas wird in dem nachfolgenden Verfahrensabschnitt B), der Chlorwasserstoff-Aufarbeitung vereinigt. Eine zusätzliche Abtrennung von Chlorwasserstoff ist auch zwischen den einzelnen Stufen durch Entspannen in einem Flash und eine anschließende Druckerhöhung möglich.

Die Apparate-Materialien müssen den hohen Anforderungen an Beständigkeit gegenüber Chlorwasserstoff und Phosgen bei hohen Temperaturen entsprechen und werden vorzugsweise ausgewählt aus der Gruppe der Werkstoffe Schwarzer Stahl, Edelstahl, Stahl-Legierungen, Nickel-Basislegierungen (z.B. Hastelloy C), Keramik, Graphit, Email-beschichtete Materialien, PTFEverkleidete Materialien.

In dem Verfahrensabschnitt B), der Chlorwasserstoff-Aufarbeitung, wird die in der Reaktion A) entstandene Gasphase gesammelt, von dem Hauptprodukt Chlorwasserstoff-Gas abgetrennt, und gegebenenfalls für eine weitere Umsetzung zu Diarylcarbonat unterzogen und in die Reaktion zugeführt. Das Hauptprodukt Chlorwasserstoff kann zur Erhöhung der Reinheit destilliert werden. Die Kombination aus wenigstens zwei Reinigungsmaßnahmen, einer Destillation in Kombination mit einer weiteren Reinigungsmaßnahme, ausgewählt aus der Gruppe bestehend aus Kondensation und Wäsche, bietet eine sehr wirtschaftliche Verfahrensführung, die dennoch zu exzellenten Reinheitsgraden führt, wobei die Reihenfolge der Reinigungsmaßnahmen beliebig ist.

Bevorzugt wird die Destillation (D) zuletzt durchgeführt, wobei hier beliebige Kombinationen möglich sind, wie beispielsweise Wäsche (W)-Destillation, Kondensation (K)-Destillation, K-W-D oder K-W-K-D. Die Kombination K-W-K-D ist ganz besonders bevorzugt.

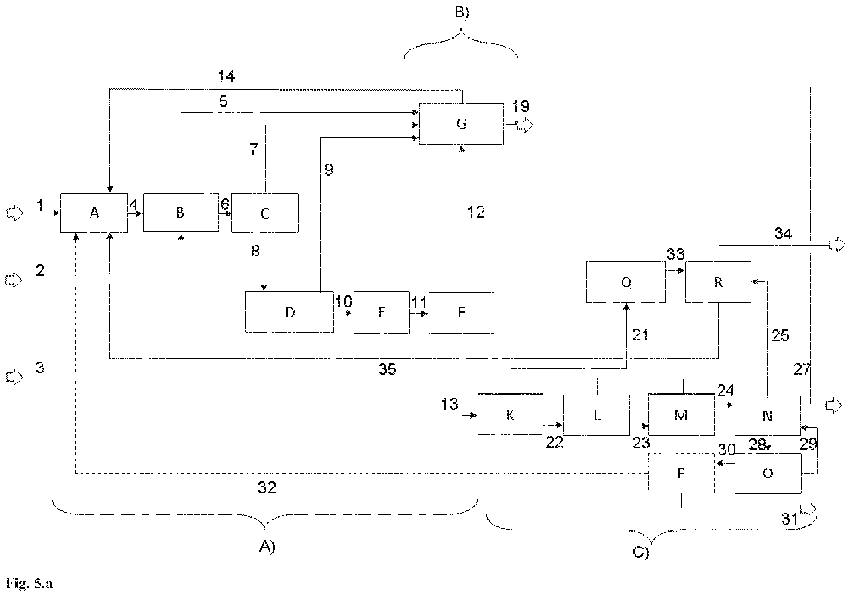

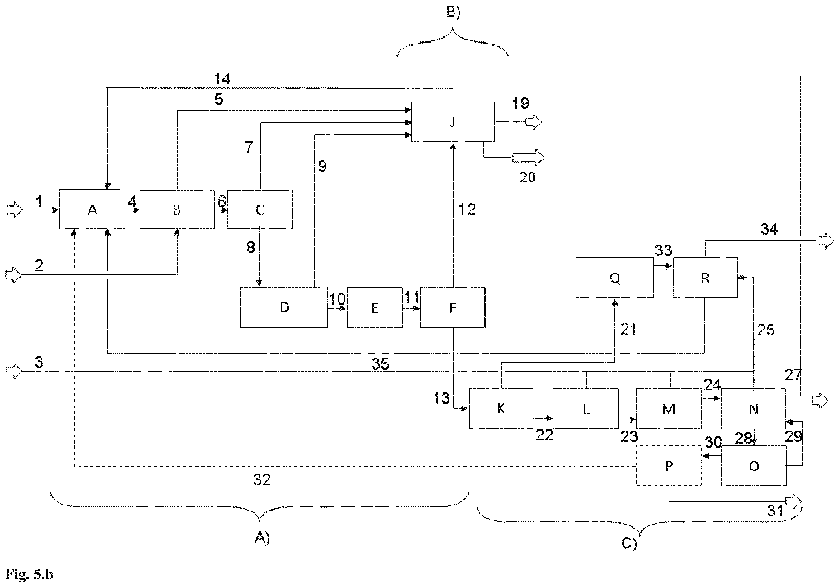

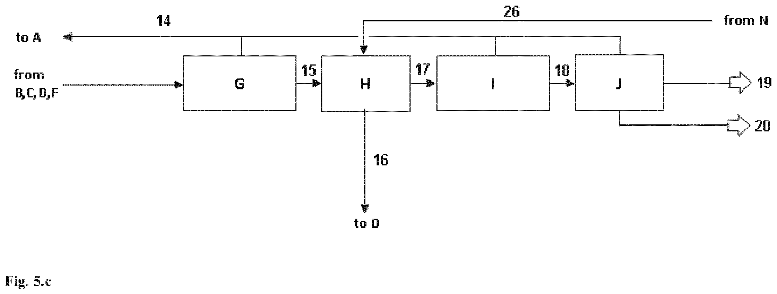

Eine graphische Übersicht dieses Verfahrens-Abschnitts gibt Fig. 5. (Chlorwasserstoff-Aufarbeitung)
In diesem Verfahrensabschnitt B werden mehrere Stoffströme aus dem Verfahrensabschnitt A) (Reaktion; s. Ströme 5,7 und 12) vereinigt und gemeinsam gereinigt. Hauptprodukt unter den leichtsiedenden Komponenten ist mit 90 Gew% oder mehr das Chlorwasserstoff-Gas; Nebenprodukte sind das im Überschuss eingesetzte Monophenol mit mehr als 5 Gew %, sowie Spuren an Aryl-chlorkohlensäureester, Diarylcarbonat und Phosgen und als Nebenprodukt aus dem Phosgen Spuren an Kohlenmonoxid und Tetrachlorkohlenstoff. Die Nebenprodukte können über verschiedene Schritte vom Hauptprodukt Chlorwasserstoff weitgehend abgetrennt werden, sodass ein Chlorwasserstoff-Gas mit einer Reinheit von mehr als 99,0 Gew.%, vorzugsweise von mehr als 99,9 Gew.% und einem Restgehalt an Phosgen und/oder Chlor-Kohlensäureestern von weniger als 100 Gew. ppm, vorzugsweise von weniger als 10 Gew. ppm und besonders bevorzugt von weniger als 10 Gew. ppb erhalten wird. Ebenso sollte der Gehalt an organischen Verbindungen im Chlorwasserstoff kleiner als 1000 Gew. ppm, vorzugsweise kleiner als 50 Gew. ppm sein, insbesondere der Gehalt an chlorhaltigen Kohlenwasserstoffen sollte kleiner 50 Gew. ppm sein.

Diese Aufgabe wird durch einen oder mehrere Schritte gelöst, die nachfolgend beschrieben sind. Vorzugsweise wird diese Aufgabe durch einen mehrstufigen Prozess gelöst.

In einer sogenannten ersten Kondensations-Stufe werden die Nebenprodukte mit einem höheren Siedepunkt als dem von Chlorwasserstoff bei geeigneter Temperatur auskondensiert. Dabei werden insbesondere höher siedende Komponenten, die in größerer Konzentration vorliegen, wie Monophenole und Diarylcarbonate weitgehend aus dem Chlorwasserstoff-Gas entfernt, die in die Reaktion zurückgeführt werden können. Diese Abtrennung gelingt besonders gut, wenn neben der tieferen Temperatur optional auch erhöhte Drücke angewendet werden. Bevorzugte Temperaturen in der ersten Kondensationsstufe sind mindestens 80°C, und für die Herstellung von Diphenylcarbonat besonders bevorzugt 90°C. Der Druck wird vorzugsweise in einem Bereich von 8 bis 25 bar (a) eingestellt, ein besonders bevorzugter Druck für die Herstellung von Diphenylcarbonat ist 12 bar (a). Die Kondensation der Nebenprodukte aus dem Chlorwasserstoff-Gas-Strom kann optional auch mehrstufig bei verschiedenen Temperaturen und / oder Drücken erfolgen.

Falls eine ausreichend tiefe Temperatur oder ein ausreichend hoher Druck technisch nicht oder nur schwer zu realisieren ist, kann diese erste Kondensations-Stufe auch übergangen werden, um die Nebenprodukte in einer nachfolgenden sogenannten HCl-Wasch-Stufe in einer geeigneten Apparatur mit geschmolzenem Diarylcarbonat aus dem Chlorwasserstoff-Strom heraus zu waschen. Stellt diese HCl-Waschstufe die erste Reinigungsstufe für den Chlorwasserstoff unter Umgehung der ersten Kondensationsstufe dar, so kann diese HCl-Waschstufe auch in sich mehrstufig ausgebildet sein und auf verschiedenen, fallenden Temperatur-Niveaus betrieben werden, um die Effizienz der Wäsche zu erhöhen. Dabei lösen sich insbesondere Monophenole sehr gut im Diarylcarbonat. Auch Spuren an Chlor-Kohlensäureester und Phosgen können in diesem Verfahrensschritt noch zum Diarylcarbonat umgesetzt werden, wenn das zur Wäsche eingesetzte Diarylcarbonat z. B. an einer geeigneten Stelle im nachfolgenden Verfahrens-Abschnitt C), der Diarylcarbonat-Aufarbeitung entnommen wird. Prinzipiell ist jeder Diarylcarbonat-Strom dieses Verfahrens-Abschnitts bis hin zum destillierten Diarylcarbonat für die HCl-Wasch-Stufe geeignet, vorteilhaft für die Umsetzung der genannten organischen Chlor-Verbindungen kann es sein, einen Katalysator- und Phenol-haltigen Diarylcarbonat-Strom für die HCl-Wasch-Stufe dem Verfahrens-Abschnitt C) zu entnehmen, um in kurzer Zeit die im Chlorwasserstoff-Gas noch vorhandenen organischen Chlor-Verbindungen zur Reaktion bringen zu können.

Ein solches geeignetes Diarylcarbonat ist z.B. das Sumpfprodukt Strom 13 (s. Fig. 4, Gesamtverfahren) am Ende des Verfahrens-Abschnitts A) (Reaktion), das zur weiteren Aufarbeitung der ersten Stufe des Verfahrens-Abschnitts C) (Diarylcarbonat-Aufarbeitung) zugeleitet wird. In diesem Diarylcarbonat sind ausreichende Mengen an Katalysator und an Phenol vorhanden. Alternativ-dazu kann ein destilliertes Diarylcarbonat in beliebiger Weise für die HCl-Wasch-Stufe verwendet werden, da die physikalische Löslichkeit der auszuwaschenden Nebenprodukte im DPC hinreichend hoch ist. Bevorzugt jedoch wird ein reines destilliertes Diarylcarbonat für die HCl-Waschstufe verwendet, das dem Stoffstrom 27 (s. Fig 4, Gesamtverfahren) entnommen wird. Zur Umsetzung der organischen Chlorverbindungen in der HCl-Wasch-Stufe kann anstelle des Diarylcarbonats als Waschmedium ebenso auch das Monophenol verwendet werden, da auch im Monophenol die physikalische Löslichkeit der auszuwaschenden Nebenprodukte hinreichend hoch ist. Dieses Monophenol kann z.B. ein Teilstrom des Monophenol-Edukt-Stromes sein. Falls eine Reaktion von Chlorestern oder Phosgen zu Diarylcarbonat erwünscht ist, so kann das zur Wäsche verwendete Monophenol in beliebiger Weise Katalysator enthalten. Die HCl-Wäsche mit Diarylcarbonat oder mit Monophenol wird vorzugsweise bei Temperaturen oberhalb des Schmelzpunktes des Diarylcarbonats durchgeführt; bei der Herstellung von Diphenylcarbonat wird eine Schmelzetemperatur von 95°C besonders bevorzugt. Die HCl-Wäsche kann bei Normaldruck oder bei erhöhtem Druck von 8 bis 25 bar (a) durchgeführt werden; bei der Herstellung von Diphenylcarbonat sind 12 bar (a) besonders bevorzugt.

Bei einer derartigen Wäsche kann ein Chlorwasserstoffgas mit einer Reinheit von 99,9 Gew% erhalten werden. Der Anteil an Phosgen liegt unter 50 Gew. ppm, der von Chlorameisensäureester liegt unterhalb der Nachweisgrenze und der Phenolgehalt wird bis auf unter 1 Gew. ppm verringert. Diese HCl-Wasch-Stufe ist nicht zwingend erforderlich und kann bei beliebiger Kombination anderer Verfahrens-Schritte miteinander auch umgangen werden.

Zur Erzielung hoher Reinheiten des Chlorwasserstoff-Gases ist eine Chlorwasserstoff-Destillation besonders gut geeignet. Um eine solche Destillation Energie-effizient durchführen zu können, ist die vorangehende Abkühlung des zu reinigenden Chlorwasserstoffs auf tiefere Temperaturen in einer vorgeschalteten zweiten Kondensations-Stufe sinnvoll, aber nicht zwingend erforderlich. Entfällt diese Stufe, dann ist in der nachfolgenden Chlorwasserstoff-Destillation eine entsprechend höhere Energie bei tiefen Temperaturen erforderlich. In dieser zweiten Kondensations-Stufe, die optional auch auf mehreren unterschiedlichen Temperatur- und /oder Druck-Niveaus arbeiten kann, werden die im Chlorwasserstoff-Gas noch enthaltenen Spuren an höher siedenden Nebenprodukten abgeschieden, insbesondere bei Anwendung höherer Drücke im Bereich von 8 bis 25 bar (a), bei Diphenylcarbonat bevorzugt 12 bar (a). Die Temperaturen können in Abhängigkeit von den technischen Gegebenheiten in einem sehr weiten Bereich von plus 25°C bis minus 50°C variieren. Diese zweite Kondensations-Stufe ist insbesondere dann sehr empfehlenswert, wenn die Wäsche in der HCl-Wasch-Stufe mit Monophenol durchgeführt wurde, da auf diese Weise die im HCl-Gas-Strom vorhandene Konzentration an Monophenol deutlich abgesenkt werden kann und damit die HCl-Destillation entlastet wird. Entfällt diese zweite Kondensations-Stufe, so sind die Anforderungen an den Energie-Bedarf in der HCl-Destillation entsprechend sehr viel höher. Die Kondensate können ebenfalls, wie in der ersten Kondensations-Stufe der Reaktion zugeführt werden.

Als vierte und letzte Stufe der Chlorwasserstoff-Aufarbeitung im Verfahrens-Abschnitt B) ist die Chlorwasserstoff-Destillation in einer besonders bevorzugten Ausführungsform besonders gut geeignet zur Herstellung eines hochreinen Chlorwasserstoffs. Sie sollte vorzugsweise bei erhöhtem Druck durchgeführt werden, da sonst der energetische Aufwand für das Einstellen alternativ erforderlicher hinreichend tiefer Temperaturen unverhältnismäßig hoch wäre. Sollten vorangegangene Reinigungs-Stufen bei Normal-Druck durchgeführt worden sein, so ist spätestens bei dieser Reinigungs-Stufe eine Verdichtung des Chlorwasserstoff-Stromes auf höhere Drücke von 8 bis 25 bar (a) sehr empfehlenswert; für die Herstellung von Diphenylcarbonat sind 12 bar (a) besonders bevorzugt. Unter diesen Bedingungen ist ein Chlorwasserstoffgas mit einer Reinheit von 99,95 Gew % erhältlich.

Alle der vier oben genannten Stufen der Chlorwasserstoff-Reinigung im Verfahrens-Abschnitt B) sind in der beschriebenen Reihenfolge erfindungsgemäß besonders gut zur Herstellung eines hochreinen Chlorwasserstoff-Gases geeignet. Die Einhaltung bestimmter Reihenfolgen oder die Durchführung aller Verfahrens-Stufen ist nicht zwingend erforderlich, sondern hängt ab von dem Grad der Verunreinigung des aus der Reaktion abgeschiedenen Chlorwasserstoffs und von dem gewünschten Reinheitsgrad des Chlorwasserstoff-Gases als Endprodukt. So ist es gegebenenfalls durchaus möglich, mit einzelnen Reinigungs-Stufen das gewünschte Ergebnis zu erzielen.

Werden die Eingangsstoff-Ströme aus dem Verfahrensabschnitt A) (Reaktion; s. Fig. 4 Ströme 5, 7 9 und 12) ohne vorherige Reinigung direkt der Chlorwasserstoff-Destillation zugeführt, so ist unter den gleichen Temperatur- und Druck-Bedingungen ebenfalls ein Chlorwasserstoffgas mit eine Reinheit von 99,95 Gew% erhältlich.

Eine Kombination der Reinigungsstufen ist durchaus in einer bestimmten, jedoch von oben genannter Aufzählung unabhängigen Reihenfolge zur Erzielung bestimmter Reinheitsgrade ausführbar.

Als Apparate zur Durchführung der ersten und zweiten Kondensations-Stufe sind klassische Kühlfallen mit einer für die Prozess-Bedingungen ausreichend hohen Wärmetauscher-Oberfläche und einer Vorrichtung zur Ausspeisung der Kondensate in die Reaktion geeignet. Solche Kühlfallen können auch mehrstufig ausgeführt und gegebenenfalls verschieden temperiert sein. Geeignete Apparate für die HCl-Wasch-Stufe sind insbesondere kontinuierlich betriebene Apparate, wie z.B. Blasensäulen-Kolonnen, Glockenboden-Kolonnen, Füllkörper-Kolonnen, Packungskolonnen, Kolonnen mit feststehenden Einbauten, in denen die Waschflüssigkeit von oben dem aufsteigenden Chlorwasserstoff-Gas entgegen geführt werden. Auch kontinuierlich betriebene Rührapparate, wie z.B. Mixer-Settler oder auch diskontinuierlich betriebene Rührapparate sind prinzipiell geeignet.

Die Chlorwasserstoff-Destillation kann in üblichen Destillations- oder Rektifikations-Kolonnen mit geeigneten Kolonnen-Einbauten vorgenommen werden.

Die Materialien für die oben genannten Apparate müssen den hohen Anforderungen an Beständigkeit gegenüber Chlorwasserstoff bei hohen Temperaturen entsprechen und werden vorzugsweise ausgewählt aus der Gruppe Schwarzer Stahl, Edelstahl, Stahl-Legierungen, Nickel-Basislegierungen (z.B. Hastelloy C), Keramik, Graphit, Email-beschichtete Materialien, PTFEverkleidete Materialien.

Im Verfahrens-Abschnitt C) der Diarylcarbonat-Aufarbeitung werden die in der Reaktion A) entstandenen höher siedenden Komponenten gesammelt, getrennt und gegebenenfalls in die Reaktion zurückgeführt; das Hauptprodukt wird dabei so weit gereinigt, dass ein Diarylcarbonat mit einer Reinheit von mehr als 99,0 Gew%, vorzugsweise von mehr als 99,8 Gew% erhalten wird.

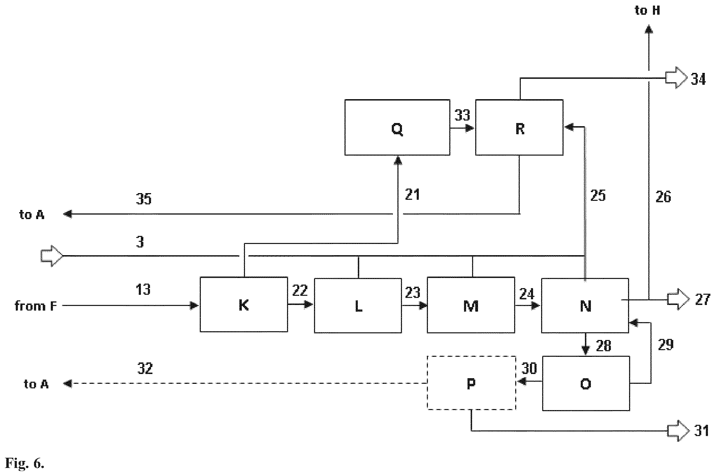

Eine graphische Übersicht dieses Verfahrens-Abschnitts gibt Fig. 6. (Diarylcarbonat-Aufarbeitung)

Dazu wird das flüssige Reaktionsprodukt, als Sumpfprodukt aus dem Verweilreaktor bzw. als Sumpfprodukt aus der optional darauf folgenden Flashstufe des Verfahrens-Abschnitts A) in die erste Destillations-Stufe des Verfahrens-Abschnitts C) überführt. Das flüssige Reaktionsprodukt enthält als Hauptprodukt das Diarylcarbonat und als wesentliche Nebenkomponente Monophenol, sowie wenige Gew% an Katalysator und an Chlorwasserstoff und wird in der ersten Destillations-Stufe bei Temperaturen von max. 250°C , bevorzugt von 180°C bis 210°C und Drücken von 0,5 bis 3,0 bar (a), vorzugsweise von 0,8 bis 1,0 bar (a) von Chlorwasserstoff befreit. Dieser Chlorwasserstoff wird der ersten Kondensations-Stufe im Verfahrens-Abschnitt B), der Chlorwasserstoff-Aufarbeitung zugeführt.

Anstelle einer Destillation als erster Verfahrens-Stufe kann auch eine Monophenol-Flash-Verdampfung verwendet werden, die gegebenenfalls auch mehrstufig ausgeführt sein kann. Aufgabe dieser Flash-Verdampfung, die in einer bevorzugten Ausführungsform die zweite Verfahrens-Stufe darstellt, ist die weitgehende Abtrennung von Phenol aus dem Hauptprodukt Diarylcarbonat, um die nachfolgende Monophenol-Destillations-Stufe zu entlasten. Unter den Flash-Bedingungen von 150°C bis 250°C, vorzugsweise von 170°C bis 200°C bei einem Druck von 0,1 bis 1,0 bar (a), vorzugsweise 0,2 bis 0,5 bar (a) wird neben der Verdampfung von Monophenol auch eine weitgehende Verdampfung des Chlorwasserstoffs bewirkt. Die abgeschiedenen Monophenol-Brüden können direkt ohne weitere Reinigung in die Reaktion zurückgeführt werden. Chlorwasserstoff-Gas wird der ersten Kondensations-Stufe im Verfahrens-Abschnitt B), der Chlorwasserstoff-Aufarbeitung zugeführt.

Anstelle der Flash-Verdampfung, als zweiter Verfahrensstufe nach der ersten Destillations-Stufe zur Entfernung von Chlorwasserstoff, kann auch eine zweite klassische Destillation des Sumpfproduktes aus der ersten Destillations-Stufe zur weitgehenden Entfernung von Monophenol unter ähnlichen Bedingungen, wie oben für die Flash-Verdampfung beschrieben, erfolgen. Es ist erstrebenswert, spätestens bis zur zweiten Verfahrens-Stufe im Verfahrens-Abschnitt C) das flüssige Diarylcarbonat-Sumpfprodukt unabhängig von der Art der Verdampfungstechnik vollständig von Spuren an Chlorwasserstoff zu befreien.

Das Sumpfprodukt der zweiten Verfahrens-Stufe, das frei von Chlorwasserstoff ist und nur noch wenig Monophenol sowie Katalysator-Bestandteile enthält, wird in der nachfolgenden dritten Verfahrensstufe, der Monophenol-Destillation, von leichtsiedenden Nebenprodukten, im wesentlichen Monophenol, fast vollständig befreit. Die Destillation erfolgt in üblichen Destillations- oder Rektifikations-Kolonnen bei Sumpftemperaturen von bis zu 250°C, vorzugsweise von 180°C bis 210°C und einem Druck von weniger als 50 mbar (a), vorzugsweise bei 10 bis 30 mbar (a). Diese Monophenol-Brüdenströme können ohne weitere Reinigung direkt der Reaktion zugeführt werden.

Dieses Sumpfprodukt aus der dritten Verfahrens-Stufe wird in der anschließenden vierten Verfahrens-Stufe, der Diarylcarbonat-Destillation, bei Sumpf-Temperaturen von max. 210°C und einem Druck von weniger als 15 mbar (a) von Schwersiedern, wie z.B. den KatalysatorBestandteilen, durch Destillation befreit. Bei einer solchen Destillation kann das hochreine Diarylcarbonat mit einem Gehalt von mehr als 99,5 Gew%, vorzugsweise von mehr als 99,8 Gew% z.B. im Seitenstrom oder über Kopf der Destillationskolonne entnommen werden. Das so gewonnene Diarylcarbonat als Hauptprodukt des Herstell-Verfahrens enthält noch Spuren an ortho-Hydroxy-Benzoesäurederivaten in Mengen von weniger als 0,5 Gew%, vorzugsweise von weniger als 0,2 Gew%, da wegen der geringen Siedepunktsdifferenzen zum Diarylcarbonat eine vollständige destillative Abtrennung dieser Nebenprodukte vom Hauptprodukt technisch sehr schwierig ist. Die Konzentration dieser Nebenprodukte im destillierten Diarylcarbonat verringert sich durch Komplex-Bildung z.B. mit Titan-Katalysatoren, wodurch diese Nebenprodukte dann als Hochsieder im Destillations-Sumpf verbleiben. Am Kopf dieser Kolonne werden noch Spuren an Leichtsiedern abgeführt und der Reaktion zugeleitet. Bei den bevorzugten Sumpf-Temperaturen von maximal 210°C zur Vermeidung von Zersetzungsreaktionen in größerem Ausmaß im Kolonnensumpf verbleiben gegebenenfalls nicht unerhebliche Mengen an Diarylcarbonat im Sumpf der Kolonne zurück, die die Ausbeute an Diarylcarbonat mindern.

Daher kann der Sumpf der Diarylcarbonat-Destillations-Kolonne optional einer fünften Verfahrensstufe, dem Diarylcarbonat-Verdampfer, zugeführt werden, in dem nach dem Prinzip von Dünnschicht-Verdampfern bei kurzen Verweilzeiten, einem Vakuum von vorzugsweise weniger als 15 mbar (a) und bei Temperaturen von mehr als 210°C eine ausreichende Menge Diarylcarbonat aus dem Sumpfprodukt der vierten Verfahrens-Stufe zurückgewonnen wird. Dieses so zurückgewonnene Diarylcarbonat, das noch Spuren an höher siedenden Nebenprodukten enthalten kann, wird vorzugsweise für eine weitere Reinigung in die Diarylcarbonat-Destillations-Kolonne der vierten Verfahrensstufe zurückgeführt. Das jetzt noch verbleibende Sumpfprodukt in der fünften Verfahrensstufe enthält zum großen Teil Katalysator-Bestandteile, die je nach eingesetztem Katalysator noch katalytisch aktiv sind und direkt oder nach einer weiteren Aufarbeitung erneut verwendet und ganz oder teilweise in die Reaktion zurückgeführt werden können. Bei Verwendung von Titan-tetrachlorid als Katalysator bildet sich das Titan-tetra-Monophenolat bzw. der gemischte Ester mit den ortho-Hydroxy-Benzoesäurederivaten, die als Nebenprodukte entstehen und ebenfalls an das Titan gebunden werden und als Hochsieder im Sumpf der Destillationen verbleiben.

Alle fünf der oben genannten Stufen der Diarylcarbonat-Aufarbeitung im Verfahrens-Abschnitt C) sind in der beschriebenen Reihenfolge erfindungsgemäß besonders gut zur Herstellung eines hochreinen Diarylcarbonats geeignet. Die Einhaltung bestimmter Reihenfolgen oder die Durchführung aller Verfahrens-Stufen ist aber nicht zwingend erforderlich, sondern hängt ab von dem Anteil an Überschüssen des Monophenols, der Art des Katalysators und der Menge an Nebenprodukten in dem aus der Reaktion erhaltenen Reaktionsgemisch, sowie von dem gewünschten Reinheitsgrad des Diarylcarbonats als Endprodukt. So ist es gegebenenfalls durchaus möglich, mit einzelnen Reinigungs-Stufen oder einer einzigen Reinigungs-Stufe das gewünschte Ergebnis zu erzielen. Auch hier ist eine Kombination der Reinigungsstufen durchaus in einer bestimmten, jedoch von oben genannter Aufzählung unabhängigen Reihenfolge zur Erzielung bestimmter Reinheitsgrade ausführbar.

Geeignete Destillations-Apparate im Verfahrens-Abschnitt C) der Diarylcarbonat-Aufarbeitung sind z.B. Glockenboden-Kolonnen, Füllkörper-Kolonnen, Packungskolonnen, Trennwandkolonnen, Kolonnen mit geeigneten Einbauten zur Erhöhung der Flüssigkeits-Oberfläche, DünnschichtVerdampfer oder Flash-Verdampfer.

Der Unterdruck für die Destillationsstufen wird üblicherweise durch Vakuum-Flüssigkeitsringpumpen erzeugt. Er kann ebenso auch durch Dampfstrahler erzeugt werden, die mit dem in diesem Verfahren erzeugten Diarylcarbonat oder mit dem in diesem Verfahren verwendeten Phenol betrieben werden.
- **Fig. 1**: Einfluss der Salol-Konzentration auf die Farbzahl des Polycarbonats
- **Fig**. **2**: zeigt diese Zusammenhänge recht deutlich für den Einsatz von zwei verschiedenen Mengen an Titan-tetrachlorid bei der Synthese von Diphenylcarbonat aus Phenol und Phosgen.
- **Fig**. **3**: zeigt die Löslichkeit von Phosgen in Phenol.
- **Fig**. **4**: Übersicht über das Gesamt-Verfahren.
- **Fig**. **5**.**a**: zeigt eine graphische Übersicht der Chlorwasserstoff-Aufarbeitung, 2-stufige Kondensation
- **Fig**. **5**.**b**: zeigt eine graphische Übersicht der Chlorwasserstoff-Aufarbeitung (nur Destillation)
- **Fig**. **5.c**: Chlorwasserstoff-Aufarbeitung (alle Stufen)
- **Fig**. **6**: zeigt eine graphische Übersicht der Diarylcarbonat-Aufarbeitung (Schritt C)

In den Figuren 4, 5.a und 5.b haben die Buchstaben mit Klammer folgende Bedeutung:
A) Ist der Verfahrens-Abschnitt "Reaktion"
B) Ist der Verfahrens-Abschnitt "Chlorwasserstoff-Aufarbeitung"
C) Ist der Verfahrens-Abschnitt "Diarylcarbonat-Aufarbeitung".

Die Buchstaben in den rechteckigen Kästen haben die folgende Bedeutung:
- A:: Edukt-Mischaggregat
- B:: Hauptreaktor
- C:: Entgasungsstufe
- D:: Nachreaktor
- E:: Verweilreaktor
- F:: Entgasungs-oder Flashstufe
- G:: Erste Kondensations-Stufe
- H:: Chlorwasserstoff-Wäsche
- I:: Zweite Kondensationsstufe
- J:: Chlorwasserstoff-Destillation
- K:: Erste Destillation (Leichtsieder-Destillation)
- L:: Flash-Verdampfung
- M:: Monophenol-Destillation
- N:: Diarylcarbonat-Destillation
- O:: Diarylcarbonat-Verdampfer (Dünnfilm-Verdampfer)
- P:: (optional) Katalysator-Lösungs-Vorlage
- Q:: Kondensator
- R:: Leichtsieder-Wäsche mit Phenol.

Die Zahlen an den Linien bezeichnen die folgenden Stoffströme:
- 1:: Phosgen
- 2:: Frisch-Katalysator
- 3:: Frisch-Monophenol
- 4:: Monophenol-Phosgen-Gemisch
- 5:: Chlorwasserstoff + Leichtsieder
- 6:: Reaktionsgemisch
- 7:: Chlorwasserstoff + Leichtsieder
- 8:: Reaktionsgemisch
- 9:: Chlorwasserstoff + Leichtsieder
- 10:: Reaktionsgemisch
- 11.: Reaktionsgemisch
- 12:: Chlorwasserstoff + Leichtsieder
- 13:: Reaktionsgemisch
- 14:: Monophenol-Kondensat
- 15:: Chlorwasserstoff
- 16:: Abfluss von Wasch-DPC
- 17:: Chlorwasserstoff
- 18:: Chlorwasserstoff
- 19:: Chlorwasserstoff-Endprodukt
- 20:: Sumpf-Produkt der Chlorwasserstoff-Destillation
- 21:: Leichtsieder-Brüden-Strom
- 22:: Diarylcarbonat
- 23:: Diarylcarbonat
- 24:: Diarylcarbonat
- 25:: Leichtsieder-Brüden
- 26:: Diarylcarbonat
- 27:: Diarylcarbonat-Endprodukt
- 28:: Diarylcarbonat-Sumpfprodukt
- 29:: Diarylcarbonat-Kondensat
- 30:: Katalysator-Salz in Diarylcarbonat-Sumpfprodukt
- 31:: Diarylcarbonat-Sumpf-Ausschleusung
- 32:: Katalysator-Lösungs-Rückführung.

### Beispiele:

### Bestimmung Salol-Gehalt

Salol (o-Hydroxy-Benzoesäurephenylester) wurde in den Diphenylcarbonat-Proben bei den Beispielen unter A) gaschromatographisch nach Hydrolyse des Titan-Salol-Komplexes mit Wasser vor der Destillation des Diphenylcarbonats bestimmt. Auf diese Weise kann die gesamte gebildete Menge des Nebenprodukts Salol im Diphenylcarbonat erfasst werden; andernfalls verbleibt ein Teil des gebildeten Salols an dem Titankomplex gebunden und entzieht sich damit der Erfassung als Nebenprodukt im Diphenylcarbonat nach dessen Destillation.

### YI

Die Bestimmung der optischen Eigenschaften der erfindungsgemäßen Formmassen geschieht durch Messung des sog. Yellowness-Index (YI) an Normprüfkörpern (60 x 40x 4 mm) nach ASTM E313. Die Herstellung der Normprüfkörper erfolgte bei einer Massetemperatur von 300 °C und einer Werkzeugtemperatur von 90 °C.

### A) Einfluss der Titan-Katalysatorrückführung auf die Bildung von Salol (o-OH-Bezoesäurephenylester) als Nebenprodukt in Diphenylcarbonat:

### Beispiel 1.

In einem 250ml Wellenbrechtopf mit Gaseinleitungsfritte (2 = 40-100 µm), Magnetrührer, Sumpf-Thermofühler, Intensivrückflußkühler, Septum für Probenahme und Ölheizbad mit Thermosteuerung wurden 141,2 g (1,5 Mol) Phenol und 355,7 mg (1,875 mMol) Titantetrachlorid (0,125 Mol% bez. auf Phenol) als Katalysator vorgelegt und auf 160°C aufgeheizt. Über einen Zeitraum von 2 Stunden wurden bei einer Sumpftemperatur von 160°C 88g (0,89 Mol) Phosgen (Molverhältnis: Phenol zu Phosgen wie 1,68 zu 1) über einen Massendurchflußmesser eingeleitet. Anschließend wurde die Gaseinleitungsfritte noch 2h mit N2-Gas gespült und die Sumpftemperatur von 160°C langsam auf 90°C abgesenkt. Danach wurde durch das Septum eine Probe für GC-Reinheits-Bestimmung gezogen. Danach wurde mit wenig N2-Begasungnochmals 1h bei 90°C gerührt und dann eine 2. Kontrollprobe für die GC-Analyse gezogen. Danach wurde das Reaktionsgemisch im Vakuum von weniger als 30 mbar (a) und einer Sumpftemperatur von 210°C destilliert, wobei Leichtsieder und Diphenylcarbonat abdestilliert wurden. Das Katalysator-haltige Sumpfprodukt wurde für Folgeversuche wieder eingesetzt, um die Wiederverwendbarkeit dieser Katalysator-Zusammensetzung zu testen.

Die GC-Proben wurden vor der Messung bei RT gründlich mit Wasser geschüttelt, um Salol aus Komplexbindungen mit Titan frei zu setzen.

Salol-Gehalt bezogen auf DPC:0,4 Gew%.

### Beispiel 2.

Wie Beispiel 1. aber mit 177,8 mg (0.937 mMol) Titantetrachlorid (0,0625 Mol% bez. auf Phenol); Salol-Gehalt bezogen auf DPC:0,3 Gew%.

### Beispiel 3., 1. Katalysator-Rückführung

Wie Beispiel 1. aber mit Einsatz des Katalysator-haltigen Destillations-Sumpfes und nach Aufstockung des Katalysator-Gehaltes mit frischen Titantetrachlorid auf einen Gesamtgehalt an Titan-Katalysator von 1,875 mMol (0,125 Mol% bez. auf Phenol);
Salol-Gehalt bezogen auf DPC:0,7 Gew%.

### Beispiel 4. bis 7., 2. bis 5. Katalysator-Rückführung

Wie Beispiel 3. mit wiederholtem Einsatz des Katalysator-haltigen Destillations-Sumpfes und nach Aufstockung des Katalysator-Gehaltes mit frischen Titantetrachlorid auf einen Gesamtgehalt an Titan-Katalysator von 1,875 mMol (0,125 Mol% bez. auf Phenol);
Salol-Gehalt bezogen auf DPC bei 2. Katalysator-Rückführung: 1,0 Gew%.
Salol-Gehalt bezogen auf DPC bei 3. Katalysator-Rückführung: 1,2 Gew%.
Salol-Gehalt bezogen auf DPC bei 4. Katalysator-Rückführung: 1,2 Gew%.
Salol-Gehalt bezogen auf DPC bei 5. Katalysator-Rückführung: 1,1 Gew%.

### Beispiel 8., 1. Katalysator-Rückführung

Wie Beispiel 2. aber mit Einsatz des Katalysator-haltigen Destillations-Sumpfes und nach Aufstockung des Katalysator-Gehaltes mit frischen Titantetrachlorid auf einen Gesamtgehalt an Titan-Katalysator von 0,937 mMol (0,0625 Mol% bez. auf Phenol);
Salol-Gehalt bezogen auf DPC:0,6 Gew%.

### Beispiel 9. bis 17., 2. bis 10. Katalysator-Rückführung

Wie Beispiel 8. mit wiederholtem Einsatz des Katalysator-haltigen Destillations-Sumpfes und nach Aufstockung des Katalysator-Gehaltes mit frischen Titantetrachlorid auf einen Gesamtgehalt an Titan-Katalysator von 0,937 mMol (0,0625 Mol% bez. auf Phenol);
Salol-Gehalt bezogen auf DPC bei 2. Katalysator-Rückführung: 0,8 Gew%.
Salol-Gehalt bezogen auf DPC bei 3. Katalysator-Rückführung: 0,9 Gew%.
Salol-Gehalt bezogen auf DPC bei 4. Katalysator-Rückführung: 0,9 Gew%.
Salol-Gehalt bezogen auf DPC bei 5. Katalysator-Rückführung: 0,9 Gew%.
Salol-Gehalt bezogen auf DPC bei 6. Katalysator-Rückführung: 0,8 Gew%.
Salol-Gehalt bezogen auf DPC bei 7. Katalysator-Rückführung: 0,8 Gew%.
Salol-Gehalt bezogen auf DPC bei 8. Katalysator-Rückführung: 0,8 Gew%.
Salol-Gehalt bezogen auf DPC bei 9. Katalysator-Rückführung: 0,7 Gew%.
Salol-Gehalt bezogen auf DPC bei 10. Katalysator-Rückführung: 0,8 Gew%.

Die Daten sind nachfolgend tabellarisch (Tab. 1) zusammengefasst:

**Tabelle 1**

| Gew% Salol in DPC als Funktion der Titantetrachlorid-Menge in mol% bezogen auf Phenol und der Anzahl an Katalysator-Rückführungen | 0,125mol% | 0,0625mol% |
|---|---|---|
| 0. Katalysator-Rückführung | 0,4 | 0,3 |
| 1. Katalysator-Rückführung | 0,7 | 0,6 |
| 2. Katalysator-Rückführung | 1,0 | 0,8 |
| 3. Katalysator-Rückführung | 1,2 | 0,9 |
| 4. Katalysator-Rückführung | 1,2 | 0,9 |
| 5. Katalysator-Rückführung | 1,1 | 0,9 |
| 6. Katalysator-Rückführung | | 0,8 |
| 7. Katalysator-Rückführung | | 0,8 |
| 8. Katalysator-Rückführung | | 0,8 |
| 9. Katalysator-Rückführung | | 0,7 |
| 10. Katalysator-Rückführung | | 0,8 |

### B) Herstellung des Polycarbonats in der Schmelze

Aus einer Vorlage wurden 15927 kg/h Schmelzegemisch, bestehend aus 7798 kg DPC/h (36402mol/h) und 8128 kg einer BPA/Phenol-Mischung/h, unter Zusatz von 6,35 kg/h Katalysatorlösung durch einen Wärmetauscher gepumpt, auf 190 °C erwärmt und durch eine Verweilkolonne geführt. Die mittlere Verweilzeit betrug 45 min. Die Katalysatormischung bestand aus 0,52 kg des Phenoladduktes von Tetraphenylphosphoniumphenolat mit Phenol (enthalten 65,5 Gew.-% Tetraphenylphosphoniumphenolat 0,786 mol) gelöst in 4,5 kg Phenol. Die BPA/Phenolmischung bestand aus 92,66 Gew.-% BPA (32990 mol) und 7,34 Gew.-% Phenol (6339 mol). Das DPC/BPA-Verhältnis beträgt 1,103 (mol/mol).

Die Schmelze wurde dann über ein Entspannungsventil in einen unter 236 mbar stehenden Abscheider geleitet. Die abfließende Schmelze wurde in einen ebenfalls unter 236 mbar stehenden Fallfilmverdampfer wieder auf 190 °C erwärmt und in einer Vorlage aufgefangen. Nach einer mittleren Verweilzeit von 30 min wurde die Schmelze in die nächsten zwei gleichartig aufgebauten Stufen gepumpt. Bedingungen der 2./3. Stufe sind 95 / 50 mbar; 227 / 273 °C und 35 / 40 Minuten.

Das entstandene Oligomer wurde in einem sich anschließenden Korbreaktor (Reaktor 1) bei einem Druck von 7 mbar und einer Temperatur von 274 °C und einer mittleren Verweilzeit von 120 min weiter aufkondensiert. Anschließend wurde die Schmelze in einem zweiten Korbreaktor (Reaktor 2) bei 290 °C und einem Druck von 0,8 mbar und einer mittleren Verweilzeit von 120 min weiter aufkondensiert, aus dem Reaktor ausgetragen und granuliert.

Um den jeweiligen Einfluss des Salols auf die Farbzahl YI zu untersuchen, wurde entsprechend eine Salolmenge in das Schmelzegemisch dazugegeben.

Die Ergebnisse sind in Tab. 2 sowie in Fig. 1 zusammengefasst.

**Tabelle 2**

| Salol | Farbzahl |
|---|---|
| Gew.-% | |
| 0 | 0,37 |
| 0,01 | 0,4 |
| 0,05 | 0,42 |
| 0,1 | 0,44 |
| 0,5 | 0,48 |
| 1 | 0,63 |
| 2,5 | 1 |
| 5 | 1,5 |

### C) Verfahren zur Herstellung von reinem Chlorwasserstoffgas:

Die nachfolgenden Prozess-Schemata verdeutlichen den Ablauf des erfinderischen GesamtVerfahrens am Beispiel der Diphenylcarbonat-Herstellung aus Phenol und Phosgen in einer besonders bevorzugten Ausführungsform ohne die Erfindung auf diesen Verfahrens-Ablauf einzuschränken. Mit den Fig. 4 (Abschnitt B)), sowie Fig. 5.a und 5.b werden besonders die Verfahrens-Schritte zur Reinigung des Chlorwasserstoffgases beschrieben.

In einer besonders bevorzugten Ausführungsform, die in Fig. 4.(Abschnitt B)) dargestellt ist, werden die Eingangsstoffströme 5, 7, 9 und 12 aus dem Verfahrensabschnitt A) der Reaktion, die jeweils mehr als 80 Gew% an Chlorwasserstoff (HCl-Gas) enthalten, in einer ersten Kondensationsstufe G vereinigt. Dort wird das HCl-Gas vom größten Teil der unter den T- und p-Bedingungen kondensierbaren Nebenprodukte befreit, die in die Reaktion zurückgeführt werden. Der so gereinigte HCl-Gas-Strom 15 wird in der HCl-Wäsche H mit einem DPC-Strom 26 gewaschen, der als Nebenprodukt-beladener DPC-Strom 16 der Reaktion zugeleitet wird, während das so gereinigte HCl-Gas 17 der zweiten Kondensationsstufe I zugeführt wird. Nach dem Entfernen der hier noch kondensierbaren Bestandteile, insbesondere DPC, wird das so gereinigte HCl-Gas 18 zur Endreinigung der HCl-Destillation J zugeführt. Daraus erhält man das hochreine HCl-Gas 19 als Endprodukt des Verfahrens-Abschnitts B), der HCl-Aufarbeitung. Der Stoffstrom 20 tritt nur in Erscheinung für den Fall, dass die HCl-Destillation die einzige Aufarbeitungsstufe darstellt.

In einer verkürzten nicht-beanspruchten Ausführungsform der HCl-Gas-Aufarbeitung, die in Fig. 5.a wiedergegeben ist, werden die Eingangsstoffströme 5, 7, 9 und 12 aus dem Verfahrensabschnitt A) der Reaktion, die jeweils mehr als 80 Gew% an Chlorwasserstoff enthalten, in der ersten Kondensationsstufe G vereinigt, die hier zweistufig ausgebildet ist und in der ersten Stufe bei 90°C und in der zweiten Stufe bei 50°C arbeitet. Das daraus erhaltene HCl-Gas 19 ist das Endprodukt dieser Reinigung, die Zusammensetzung ist der Fig. 5.a zu entnehmen.

Eine andere verkürzte, nicht beanspruchte Ausführungsform der HCl-Gas-Aufarbeitung ist in Fig.5.b wiedergegeben. Hier werden die Eingangsstoffströme 5, 7, 9 und 12 aus dem Verfahrensabschnitt A) der Reaktion, die jeweils mehr als 80 Gew.-% an Chlorwasserstoff enthalten direkt der HCl-Destillation J zugeleitet. Dabei wird als Endprodukt dieser Reinigung das HCl-Gas (19) mit einer Reinheit > 99,95 Gew.-% erhalten sowie Mengen an Phosgen < 10 Gew.-ppm und an organischen Verbindungen < 10 Gew.-ppm.

Tabelle 3 zeigt, dass eine Kondensation oder eine Wäsche nicht ausreichend sind, um eine ausreichende Reinheit in Bezug auf Phosgen im HCl zu erreichen. Eine Destillationsstufe ist erforderlich.

Zudem wurden weitere Kombinationen an Reinigungsmaßnahmen geprüft und deren Reinigungs-Effizienz mit den Energieverbräuchen verschiedener Energieniveaus in Relation gebracht. Auch hier ist es von großem wirtschaftlichem Interesse, möglichst wenig Kälteenergie auf niedrigem Temperaturniveau (eg. -40°C) zu verwenden, da diese Energieniveaus deutlich unwirschaftlicher in der Erzeugung sind als höhere Energieniveaus (eg. 30°C).

Tabelle 3 zeigt an dieser Stelle, dass eine Kombination aus einer Destillation mit wenigstens einer weiteren Reinigungsmaßnahme entweder zu einer Reduzierung des Verbrauchs an Kühlmittel oder zu einer verbesserten Nutzung von Kühlmittel mit erhöhtem Temperaturniveau, wodurch die Nutzung von kostspieligem Kühlmittel mit niedrigem Temperaturniveau (eg. -40°C) deutlich reduziert werden kann.

**Tabelle 3**

| | **Reinheiten** | | | | **Energie** | | |
|---|---|---|---|---|---|---|---|
| | Gew. % | Gew. ppm | | | kW | | |
| | HCl | Phosgen | Phenol | DPC | Energie Kühlmittel (30°C) | Energie Kühlmittel (6°C) | Energie Kühlmittel (-40°C) |
| W* | | 20000 | | | | | |
| K* | | 15000 | | | | | |
| D* | 99,95 | 1 | 0 | 0 | 0 | 0 | 178 |
| W-D | 99,95 | 1 | 0 | 0 | 0 | 0 | 151 |
| K-D | 99,95 | 7 | 0 | 0 | 376 | 47 | 38 |
| K-W-D | 99,95 | 1 | 0 | 0 | 284 | 0 | 44 |
| K-W-K-D | 99,95 | 1 | 0 | 0 | 370 | 44 | 38 |

| | | | | | | | |
|---|---|---|---|---|---|---|---|
| Kondensation K Wäsche W Destillation D * : Vergleichsbeispiel | | | | | | | |

## Patentansprüche

1. Verfahren zur Herstellung von Diarylcarbonat und gasförmigem Chlorwasserstoff aus Monohydroxy-aromaten und Phosgen in der Schmelze in Gegenwart von wenigstens einem Katalysator und ohne Zugabe von zusätzlichem Lösemittel, **dadurch gekennzeichnet, dass** der gebildete gasförmige Chlorwasserstoff zur Entfernung der Nebenprodukte wenigstens einer Destillation und wenigstens einer weiteren Reinigungsmaßnahme ausgewählt aus der Gruppe bestehend aus Kondensation und Wäsche unterworfen wird.

2. Verfahren gemäß Anspruch 1, **dadurch gekennzeichnet, dass** der gebildete gasförmige Chlorwasserstoff zur Entfernung der Nebenprodukte zunächst einer Kondensation, dann einer Wäsche, anschließend einer Destillation unterworfen wird.

3. Verfahren gemäß Anspruch 1 oder 2, **dadurch gekennzeichnet, dass** der gebildete gasförmige Chlorwasserstoff zur Entfernung der Nebenprodukte zunächst einer Kondensation, dann einer Wäsche, anschließend einer weiteren Kondensation gefolgt von einer Destillation unterworfen wird.

4. Verfahren gemäß einem der Ansprüche 1 bis 3, **dadurch gekennzeichnet, dass** die Kondensation bei einer Temperatur von wenigstens 80°C und einem Druck von 8 bis 25 bar durchgeführt wird.

5. Verfahren gemäß einem der Ansprüche 1 bis 4, **dadurch gekennzeichnet, dass** die HCl-Destillation bei einer Temperatur von -50°C bis +25°C und einem Druck von 8 bis 25 bar durchgeführt wird.

6. Verfahren gemäß einem der Ansprüche 1 bis 5, **dadurch gekennzeichnet, dass** der aufgereinigte gasförmige Chlorwasserstrom wenigstens zum Teil einer elektrochemischen und / oder einer thermischen Oxidation unterworfen wird.

## Claims

1. Process for preparing diaryl carbonate and gaseous hydrogen chloride from monohydroxyaromatics and phosgene in the melt in the presence of at least one catalyst and without addition of additional solvent, **characterized in that** the by-products have been removed from the gaseous hydrogen chloride formed by subjecting it to at least one distillation and at least one further purification measure selected from the group consisting of condensation and scrubbing.

2. Process according to Claim 1, **characterized in that** the by-products have been removed from the gaseous hydrogen chloride formed by subjecting it firstly to a condensation, then to a scrubbing, and subsequently to a distillation.

3. Process according to Claim 1 or 2, **characterized in that** the by-products have been removed from the gaseous hydrogen chloride formed by subjecting it firstly to a condensation, then to a scrubbing, subsequently to a further condensation followed by a distillation.

4. Process according to any of Claims 1 to 3, **characterized in that** the condensation is performed at a temperature of at least 80°C and a pressure of 8 to 25 bar.

5. Process according to any of Claims 1 to 4, **characterized in that** the HCl distillation is performed at a temperature of -50°C to +25°C and a pressure of 8 to 25 bar.

6. Process according to any of Claims 1 to 5, **characterized in that** the purified gaseous chlorine water stream is subjected at least partly to an electrochemical and/or thermal oxidation.

## Revendications

1. Procédé pour la préparation de carbonate de diaryle et d'acide chlorhydrique gazeux à partir de monohydroxyaromatiques et de phosgène en masse fondue en présence d'au moins un catalyseur et sans addition de solvant supplémentaire, **caractérisé en ce que** l'acide chlorhydrique gazeux formé est soumis à au moins une distillation et à au moins une autre mesure de purification, choisie dans le groupe constitué par la condensation et le lavage, pour éliminer les produits secondaires.

2. Procédé selon la revendication 1, **caractérisé en ce que** l'acide chlorhydrique gazeux formé est d'abord soumis à une condensation, puis à un lavage et ensuite à une distillation pour éliminer les produits secondaires.

3. Procédé selon la revendication 1 ou 2, **caractérisé en ce que** l'acide chlorhydrique gazeux formé est d'abord soumis à une condensation, puis à un lavage, ensuite à une autre condensation, suivie d'une distillation pour éliminer les produits secondaires.

4. Procédé selon l'une quelconque des revendications 1 à 3, **caractérisé en ce que** la condensation est réalisée à une température d'au moins 80°C et à une pression de 8 à 25 bars.

5. Procédé selon l'une quelconque des revendications 1 à 4, **caractérisé en ce que** la distillation de HCl est réalisée à une température de -50 à +25°C et à une pression de 8 à 25 bars.

6. Procédé selon l'une quelconque des revendications 1 à 5, **caractérisé en ce que** le flux d'acide chlorhydrique gazeux purifié est soumis au moins en partie à une oxydation électrochimique et/ou à une oxydation thermique.
